# EUROPEAN PATENT SPECIFICATION

(11) **EP 1 720 989 B1**
(45) Date of publication and mention of the grant of the patent: **13.08.2008**
(21) Application number: 05720309.3
(22) Date of filing: 02.03.2005
(51) Int. Cl.: C12N 15/82, A01H 1/00

(54) **METHOD FOR IMPROVING PRODUCTIVITY OF PLANT BY CHLOROPLAST TECHNOLOGY**
VERFAHREN ZUR VERBESSERUNG DER PRODUKTIVITÄT EINER PFLANZE DURCH CHLOROPLASTENTECHNOLOGIE
PROCEDE POUR AMELIORER LA PRODUCTIVITE DE PLANTES PAR TECHNOLOGIE CHLOROPLASTE

(30) Priority: 03.03.2004 JP 2004059513
(43) Date of publication of application: 15.11.2006
(73) Proprietor: National University Corporation Nara Institute of Science and Technology, Ikoma-shi, Nara 630-0192 (JP); Kinki University, Higashiosaka-shi Osaka 577-8502 (JP)
(72) Inventor: YOKOTA, Akiho c/o Nat. Univ. Corp. Nara Institute, Ikoma-shi Nara 630-0192 (JP); SHIGEOKA, Shigeru, Sakai-shi Osaka 591-8022 (JP); TOMIZAWA, Ken-ichi c/o Res. Inst. of Innovative, Soraku-gun Kyoto 619-0292 (JP)
(74) Representative: Kalhammer, Georg
(86) International application number: PCT/JP2005/004037
(87) International publication number: WO 2005/085454

(56) References cited:
- EP-A- 1 036 842
- WO-A-98/18940
- MIYAGAWA YOSHIKO ET AL: "Overexpression of a cyanobacterial fructose-1,6-/sedoheptulose-1,7-bi sphosphatase in tobacco enhances photosynthesis and growth." NATURE BIOTECHNOLOGY, vol. 19, no. 10, October 2001 (2001-10), pages 965-969, XP002344765 ISSN: 1087-0156
- MALIGA P: "TOWARDS PLASTID TRANSFORMATION IN FLOWERING PLANTS" TRENDS IN BIOTECHNOLOGY, ELSEVIER PUBLICATIONS, CAMBRIDGE, GB, vol. 11, no. 3, March 1993 (1993-03), pages 101-107, XP000892855 ISSN: 0167-7799
- SEUTER ANJA ET AL: "Overexpression of the potential herbicide target sedoheptulose-1,7-bisphosphatase from Spinacia oleracea in transgenic tobacco" MOLECULAR BREEDING, vol. 9, no. 1, 2002, pages 53-61, XP002344766 ISSN: 1380-3743

## Description

### Technical Field

The present invention relates to a transformed plant which has high photosynthesis activity and is excellent, particularly in fixation of carbon dioxide.

### Background Art

A plant performs photosynthesis, fixes carbon dioxide in the air, and synthesizes a sugar and an organic substance which become energy source for an organism. In a plant, a process of fixing carbon dioxide in the air and synthesizing a sugar from carbon dioxide is called the Calvin cycle. The Calvin cycle does not need light energy, and is classified into the following two stages. The first stage is a process in which 3-phosphoglyceric acid (PGA) is synthesized from ribulose-1,5-bisphosphate (RuBP) and carbon dioxide, and this is further reduced, thereby to synthesize glyceraldehyde-3-phosphate (GAP). The second stage is a process in which a part of synthesized GAP is used for synthesizing a sugar (photosynthesis product), and a remaining GAP is reproduced into RuBP via fructose-1,6-bisphosphate (FBP), fructose-6-phosphate (F6P), sedoheptulose-1,7-bisphosphate (SBP), sedoheptulose-7- phosphate (S7P), ribose-5-phosphate and the like. Thereupon, synthesis of PGA from RuBP, that is, uptake of carbon dioxide into the Calvin cycle is catalyzed by ribulose-1,5-bisphosphate carboxylase/oxygenase (hereinafter, abbreviated as Rubisco). In the second stage, aldolase [enzyme which reversibly catalyzes the reaction from GAP and dihydroxyacetone phosphate (DHAP) to FBP, and the reaction from DHAP and erythrose-4-phosphate (E4P) to SBP, respectively], fructose-1,6-bisphosphatase (FBPase; enzyme which catalyzes the reaction from FBP to F6P), sedoheptulose-1,7-bisphosphatase (SBPase; enzyme which catalyzes the reaction from SBP to S7P), and transketolase are responsible for a metabolic reaction as the rate-limiting enzyme.

Many enzymes which act in the Calvin cycle are present at a higher level than that required for maintaining a continuous reaction for fixing carbon dioxide, in some cases. However, it is known that although FBPase and SBPase are important rate-limiting enzymes in the Calvin cycle, its level is extremely lower as compared with other enzymes in the Calvin cycle (see, Miyagawa et al., Nature Biotechnology, 2001, vol.19, p.965-969).

For this reason, as a transgenic plant for enhancing photosynthesis ability, a vector having a promoter which permanently and specifically expresses a fructose-1,6-bisphosphatase gene (cy-FBPase gene) of a cytosol obtained from a mesophyll cell peculiar in plant leaves, and a transgenic plant transformed with the vector are reported (International Publication WO 98/18940).

In addition, a method for expressing FBP/SBPase derived from a cyanobacterium Synechococcus PCC7942 gene is reported. According to this method, it is known that a transformed plant has higher photosynthesis activity as compared with a wild strain, and its growth is promoted (see JP-A-253768/2000, Miyagawa et al., Nature Biotechnology, 2001, vol.19, p.965-969).

However, any of the aforementioned transformants is such that each gene is introduced into a leaf nuclear genome by introducing a plasmid constructed using a gene into Agrobacterium tumefaciens, and infecting a leaf disc with this. For this reason, a protein expressed from a gene introduced into a plant was transferred into a chloroplast with a low possibility.

In addition, introduction of a heterogeneous gene into a nuclear genome gives a fear that an introduced artificially' modified gene is diffused into the environment by crossing or mating. Further, expression of the thus introduced gene is unstable, and an expression amount, consequently, the effect is greatly different every plant.

A higher plant chloroplast is present at the number of about 100 per one cell of an adult leaf, and 100 copies of a chloroplast genome are present per one chloroplast (see Archives of Biotechnology and Biophysics, 1996, vol.334, p.27-36; Bendich, A.J. BioEssays, 1987, vol.6, p.279-282).

This means that, if one copy of a foreign gene is inserted into a chloroplast genome, 10000 copies becomes to be present per cell in a transformant, and high expression of an introduced gene can be expected due to a large copy number (see Maliga, P. Trends in biotechnology, 1993, vol. 11, p.101-107).

Further, since introduction of a gene into a chloroplast utilizes homologous recombination, positional effect seen upon insertion into anucleus is not caused, and stable gene expression is performed. In addition, since a chloroplast is maternally inherited, it is thought that introduction of a gene into a chloroplast has many advantages, such as prevention of an introduced gene from flying into the environment via pollens.

An expression vector which can highly express a desired protein in a chloroplast, a transformed chloroplast transformed using the expression vector, and a plant having the transformed chloroplast are known. This expression vector is **characterized in that** it has a psbA promoter, and a ribosome-binding site upstream of a translation initiation point of a gene encoding a protein. This method is aimed at producing a protein having pharmacological activity, and a protein useful as a material for medicine industry, using a plant instead of production with microorganisms. In the Example, expression of the protein is confirmed in plants transformed using a gene of the green fluorescent protein (see Maliga, P. Trends in biotechnology, 1993, vol 11, p. 101-107)

However, in this reference, there is no description regarding improvement in photosynthesis activity or fixation of carbon dioxide in plants, and there is no description regarding FBPase or SBPase which is the rate-limiting enzyme of the Calvin cycle, or those genes.

### Disclosure of the Invention

An object of the present invention is to produce a transformed plant which has higher photosynthesis activity as compared with the wild strain, and has promoted the growth, by expressing a gene of an enzyme involved in photosynthesis of higher plants, particularly, in the Calvin cycle. More particularly, the object is to introduce a gene of an enzyme which is rate-limiting in the Calvin cycle into chloroplast DNA, and produce a plant having transformed chloroplasts, photosynthesis ability of which is enhanced.

The present inventors have found that transformation technique which can assuredly express a protein having FBPase and/or SBPase in higher plant chloroplasts. In addition, a transformed plant not only has high photosynthesis activity, but also is grown into a plant having a greater plant body. The present invention has been completed by various further studies based on these findings.

That is, described herein is:
(1) A gene recombination vector containing an expression cassette for enhancing photosynthesis activity, comprising a DNA fragment comprising a gene encoding a protein having FBPase and/or SBPase activities between a Rubisco large subunit gene and a acetyl CoA carboxylase subunit gene,
(2) The vector according to the above (1), wherein the protein having FBPase activity is any one of the followings;
   (a) a protein comprising an amino acid sequence described in SEQ ID NO: 1 of Sequence Listing;
   (b) a protein comprising an amino acid sequence in which one or several amino acids are deleted, substituted, added or inserted in SEQ ID NO: 1 of Sequence Listing, and having FBPase activity; and
   (c) a protein having at least 60% or more homology to an amino acid sequence described in SEQ ID NO: 1 of Sequence Listing, and having FBPase activity,
(3) The vector according to the above (1), wherein the gene encoding a protein having FBPase activity is a gene comprising any one of the following DNAs;
   (a) DNA comprising a nucleotide sequence described in SEQ ID NO: 2 of Sequence Listing:
   (b) DNA comprising a nucleotide sequence in which one or several bases are deleted, substituted, added or inserted in SEQ ID NO: 2 of Sequence Listing, and encoding a protein having FBPase activity;
   (c) DNA which hybridizes with DNA comprising a nucleotide sequence complementary to DNA comprising a nucleotide sequence described in SEQ ID NO: 2 of Sequence Listing under stringent conditions, and comprises a nucleotide sequence encoding a protein having FBPase activity; and
   (d) DNA having at least 60% or more homology to DNA comprising a nucleotide sequence described in SEQ ID NO: 2 of Sequence Listing, and comprising a nucleotide sequence encoding a protein having FBPase activity,
(4) The vector according to the above (1), wherein the protein having SBPase activity is any one of the following proteins;
   (a) a protein comprising an amino acid sequence described in SEQ ID NO: 3 of Sequence Listing;
   (b) a protein comprising an amino acid sequence in which one or several amino acids are deleted, substituted, added or inserted in SEQ ID NO: 3 of Sequence Listing, and having SBPase activity; and
   (c) a protein having at least 60% or more homology to an amino acid sequence described in SEQ ID NO: 3 of Sequence Listing, and having SBPase activity,
(5) The vector according to the above (1), wherein the gene encoding a protein having SBPase activity is a gene comprising any one of the following DNAs;
   (a) DNA comprising a nucleotide sequence described in SEQ ID NO: 4 of Sequence Listing;
   (b) DNA comprising a nucleotide sequence in which one or several bases are deleted, substituted, added or inserted in SEQ ID NO: 4 of Sequence Listing, and encoding a protein having SBPase activity;
   (c) DNA which hybridizes with DNA comprising a nucleotide sequence complementary to DNA comprising a nucleotide sequence described in SDQ ID NO: 4 of Sequence Listing under stringent conditions, and comprises a nucleotide sequence encoding a protein having SBPase activity; and
   (d) DNA having at least 60% or more homology to DNA comprising a nucleotide sequence described in SDQ ID NO: 4 of Sequence Listing, and comprising a nucleotide sequence encoding a protein having SBPase activity,
(6) The vector according to (1), wherein the protein having FBPase and SBPase activities is any one of the followings:
   (a) a protein comprising an amino acid sequence described in SEQ ID NO: 5 of Sequence Listing;
   (b) a protein comprising an amino acid sequence in which one or several amino acids are deleted, substituted, added or inserted in SEQ ID NO: 5 of Sequence Listing; and having FBPase and SBPase activities; and
   (c) a protein having at least 60% or more homology to an amino acid sequence described in SEQ ID NO: 5 of Sequence Listing; and having FBPase and SBPase activities,
(7) The vector according to the above (1), wherein the gene encoding a protein having FBPase and SBPase activities is a gene comprising any one of the following DNAs;
   (a) DNA comprising a nucleotide sequence described in SEQ ID NO: 6 of Sequence Listing;
   (b) DNA comprising a nucleotide sequence in which one or several bases are deleted, substituted, added or inserted in SEQ ID NO: 6 of Sequence Listing, and encoding a protein having FBPase and SBPase activities;
   (c) DNA which hybridizes with DNA comprising nucleotide sequence complementary to a DNA comprising a nucleotide sequence described in SEQ ID NO: 6 of Sequence Listing under stringent conditions, and comprises a nucleotide sequence encoding a protein having FBPase and SBPase activities; and
   (d) DNA having at least 60% or more homology to DNA comprising a nucleotide sequence described in SEQ ID NO: 6 of Sequence Listing, and comprising a nucleotide sequence encoding a protein having FBPase and SBPase activities,
(8) The vector according to any one of the above (1) to (7), wherein the expression cassette has a ribosome-binding site upstream of the translation initiation point of the DNA fragment comprising a gene encoding a protein having FBPase and/or SBPase activities,
(9) The vector according to the above (8), wherein the expression cassette has a promoter upstream of a ribosome-binding site, and a terminator downstream of DNA fragment comprising a gene encoding a protein having FBPase and/or SBPase activities,
(10) The vector according to the above (9), wherein the promoter and the terminator are a promoter and a terminator derived from tobacco chloroplasts, respectively,
(11) The vector according to any one of the above (1) to (10), wherein the Rubisco large subunit gene and the acetyl CoA carboxylase subunit gene are genes derived from tobacco, respectively,
(12) A recombinant gene vector comprising an expression cassette containing a DNA fragment comprising a gene encoding a protein having FBPase and/or SBPase activities between a tobacco-derived Rubisco large subunit gene and a acetyl CoA carboxylase subunit gene, having a ribosome-binding site upstream of the translation initiation point of the DNA fragment, having a tobacco-derived promoter between a Rubisco large subunit gene and a ribosome-binding site, and having a tobacco-derived terminator between the acetyl CoA carboxylase subunit gene and the DNA fragment,
(13) A transformed chloroplast characterized in that the vector described in any one of the above (1) to (12) is introduced into chloroplasts,
(14) A plant containing the transformed chloroplasts in the above (13),
(15) The plant according to the above (14), wherein the plant is tobacco, and
(16) A plant having 2-fold or higher FBPase activity compared to the original one, characterized in that a FBP/SBP gene is introduced into chloroplast genome of higher plants and expressed using a chloroplast transformation technique.

Also, described herein is a process for producing a plant having transformed chloroplasts, comprising inserting a DNA fragment comprising a gene encoding a protein having FBPase and/or SBPase activities into a non-coding region between genes of chloroplast DNA.

The vector of the present invention can assuredly introduce a protein having FBPase and/or SBPase activities into higher plant chloroplasts. In a plant transformed with the vector of the present invention, since expression of a protein having FBPase and/or SBPase activities, which is a rate-limiting enzyme of the Calvin cycle, is enhanced, photosynthesis ability is enhanced as compared with the wild strain. As a result, in the transformed plant of the present invention, ability to synthesize sugars or starch can be enhanced as compared with the wild strain. In addition, the transformed plant of the present invention is tall, has a large area of leaves, has a thick stem, and the plant can grow rapidly. Therefore, cultivation of the transformed plant using the vector of the present invention can be a very effective means for producing a quickly growing, or a high yield plant.

In the transformed plant of the present invention, since a gene encoding a protein having FBPase and/or SBPase activities is introduced directly into the chloroplast genome rather than into the nuclear genome, there is no fear that the introduced gene is diffused through pollens. That is, there is no fear of environmental pollution that the pollen is scattered in a wide range via the wind or an insect, and this adversely inf luences on an animal and plant kingdom, for example, as in a plant in which a gene is introduced in a nucleus. In addition, expression is stable among transformants. In addition, the transformed plant of the present invention in which a gene is directly introduced into the chloroplast genome has enhanced ability to synthesize sugars or starches, and has a tall plant body and the large leaves compared with a plant transformed the gene into the nuclear genome, and can grow quickly with a high yield.

Since by utilizing recombinant DNA technology, photosynthesis which is the primary metabolic process in higher plants is improved, and thus their quick growth or high yields are made possible, the present invention can be an extremely important technique for responding to future food crisis.

In addition, in the transformed plant of the present invention, a rate-limiting enzyme of the Calvin cycle which plays an important role among photosynthesis, in particular, in fixation of carbon dioxide is enhanced. For this reason, since the transformed plant of the present invention has an enhanced rate of taking up carbon dioxide in the air, and can decrease the concentration of carbon dioxide in the air, cultivation of the plant can also contribute to suppression of global warming.

### Brief Description of the Drawings

Fig. 1 is a view showing an expression vector pLD200-S.7942FBP/SBPase;
Fig. 2 is a view showing confirmation of gene introduction by PCR. In the figure, W denotes a wild strain;
Fig. 3 is a view showing confirmation of the introduced gene and the expressed protein in a plant 10 weeks after seeding.
   In the figure, W stands for the wild strain;
Fig. 4 is a view showing comparison of FBPase activity in an upper leaf and a lower leaf 10 weeks and 18 weeks after seeding.
   In the figure, the ordinate axis is for FBPase activity;
Fig. 5 is a view showing photosynthesis activity 10 weeks after seeding;
Fig. 6 is a view showing a growth rate. In the figure, the ordinate axis denotes the height (cm) of a plant;
Fig. 7 is a view showing plants 18 weeks after seeding; and
Fig. 8 is a view showing stems and roots in plants 18 weeks after seeding.

### Best Mode for Carrying Out the Invention

The protein having FBPase and/or SBPase activities used in the present invention is a protein which can be a rate-limiting enzyme of the Calvin cycle. The protein may have activity of' any enzyme of FBPase or SBPase, or may have activities of both enzymes. In particular, in higher plants, a protein having enzyme activity of SBPase which can be pacemaker enzyme governing the rate of a series of reactions of the Calvin cycle as a whole, and a protein having both activities of FBPase and SBPase (hereinafter, abbreviated as FBP/SBPase) are preferable.

Examples of the protein having FBPase activity include an amino acid sequence represented by SEQ ID NO: 1. In addition, examples of the protein having SBPase activity include an amino acid sequence represented by SEQ ID NO: 3. Examples of the protein exhibiting FBP/SBPase activities include an amino acid sequence of cyanobacterium-derived FBP/SBPase represented by SEQ ID NO: 5. The protein having FBPase and/or SBPase activities used in the present invention includes proteins having an amino acid sequence in which one or several amino acids are deleted, substituted, added or inserted in the aforementioned respective amino acid sequences, and each having FBPase activity, SBPase activity or FBP/SBPase activities. Further, the protein having FBPase and/or SBPase activities used in the present invention include a protein having at least 60% or more homology, preferably 80% or more homology, more preferably 90% or more homology, and furthermore preferably 95% or more homology to an amino acid sequence described in SEQ ID NO: 1, 3 or 5, each of which having FBPase activity, SBPase activity or FBP/SBPase activities.

As used herein, "homology" regarding an amino acid sequence is used to mean an extent of coincidence of amino acid residues constituting each sequence between sequences when the primary structures of proteins are compared.

In addition, as used herein, "one or several (around 2 to 6) amino acids are deleted, substituted, added or inserted" regarding an amino acid sequence means that a naturally-occurring number of amino acids are deleted, substituted, added or inserted by the well-known technological method such as a site-specific mutagenesis method.

The DNA fragment comprising a nucleotide sequence encoding a protein having FBPase and/or SBPase activities used in the present invention refers to DNA encoding each enzyme of FBPase, SBPase, and FBP/SBPase and DNA encoding a protein having an active site of the aforementioned each enzyme. Examples of the nucleotide sequence encoding a protein having FBPase activity include a DNA sequence represented by SEQ ID NO: 2. Examples of the nucleotide sequence encoding a protein having SBPase activity include a DNA sequence represented by SEQ ID NO: 4. Examples of the nucleotide sequence encoding a protein having FBP/SBPase activities include a DNA sequence represented by SEQ I NO: 6. The DNA fragment encoding a protein having FBPase and/or SBPase activities used in the present invention includes DNA which comprises a nucleotide sequence in which one or several bases are deleted, substituted, added or inserted in the aforementioned DNA sequence represented by SEQ ID NO: 2, 4 or 6, and encodes a protein having FBPase activity, SBPase activity, or FBP/SBPase activities. As used herein, "one or several bases are deleted, substituted, added or inserted" regarding a nucleotide sequence means that a naturally-occurring number (1 to several) of bases are deleted, substituted, added or inserted by the well-known technological method such as a site-directed mutagenesis method.

The DNA fragment encoding a protein having FBPase and/or SBPase activities used in the present invention includes DNA hybridizing with DNA comprising a nucleotide sequence complementary to each of DNA sequence shown in SEQ ID NO: 2, 4 or 6 under stringent condition, which also comprises a nucleotide sequence encoding a protein having FBPase activity, SBPase activity or FBP/SBPase activities. The DNA which can hybridize under stringent conditions means DNA which is obtained by using the aforementioned DNA as the probe, by such as the colony hybridization method, the plaque hybridization method or the Southern blot hybridization method. The stringent condition refers to the hybridizing condition of SSC solution of the salt concentration about 0.1 to 2-fold (a composition of SSC solution at 1-fold concentration comprises 150 mM sodium chloride, and 15 mM sodium citrate) at the temperature of about 65°C.

Further, the DNA fragment encoding a protein having FBPase and/or SBPase activities used in the present invention include DNA having at least 60% or more homology to each DNA sequence shown in SEQ ID NO: 2, 4 or 6, and also comprising a nucleotide sequence encoding a protein having FBPase activity, SBPase activity or FBP/SBPase activities. The DNA having homology refers to DNA having at least about 60% or more homology, preferably DNA having about 80% or more homology, more preferably DNA having about 90% or more homology, and furthermore preferably DNA having about 95% or more homology, under high stringent conditions. High stringent conditions refer to, for example, conditions where the sodium concentration is about 19 to 40 mM, preferably about 19 to 20 mM, and the temperature is about 50 to 70°C, preferably about 60 to 65°C. In particular, the conditions where the sodium concentration is about 19 mM and the temperature is about 65°C is most preferable.

Hereinafter, a DNA fragment encoding a protein having FBPase and/or SBPase activities, as well as said hybridizing DNA and said DNA having homology are also referred to as the gene to be introduced.

The expression cassette of the present invention is such that a nucleotides sequence which forms a complementary base pair with a gene [ rbcL gene, accD gene, ] sequence of a chloroplast DNA is added to 5'- and 3'- side of the gene to be introduced, so that the cassette is assuredly introduced into chloroplast DNA by homologous recombination. A nucleotide sequence forming a complementary base pair can be preferably used as long as it is a sequence having a nucleotide sequence of about 500 to 1500, which has a homologous part forming a complementary base pair with a gene of chloroplast DNA. Examples of such nucleotide sequence include a sequence which is substantially the same as that of a gene of chloroplast DNA, a sequence which is substantially the same as a partial sequence of a gene of chloroplast DNA, or a nucleotide sequence complementary to a sequence containing a sequence which is substantially the same as that of a gene of chloroplast DNA.

In addition, the nucleotide sequence is not limited to the aforementioned gene sequence of chloroplast DNA as long as it has a nucleotide sequence of about 1000 to 1500 from a position in which a gene to be introduced has been introduced, and forms a complementary base pair with a gene (rbcL gene, accD gene,) of chloroplast DNA.

In this regard, it is necessary that a nucleotide sequence of chloroplast DNA is not changed except that a foreign gene is introduced. A nucleotide sequence of chloroplast DNA into which a foreign gene is introduced has been already registered in NCBI database, and is disclosed (registration number: NC 001879). A position at which a gene to be introduced is introduced in a chloroplast DNA is between rbcL gene and accD gene, and is preferably a non-coding region sufficiently apart from each gene. The sufficiently apart is at least 50 bases or more, preferably about 100 to 1000 bases, more preferably about 200 to 500 bases from a gene. The non-coding region may be any non-coding region on a chloroplast DNA.

An expression cassette using the rbcL gene and the accD gene will be explained in detail below.

The rbcL gene constituting an expression cassette which enhances photosynthesis activity is the gene of Rubisco encoded in the chloroplast genome. Rubisco catalyzes a CO₂ fixing reaction (carboxylase reaction) which is an initial stage of CO₂ fixation reaction cycle (Calvin cycle) of photosynthesis, and is a key enzyme which is rate-limiting in metabolism in the cycle. The enzyme also catalyzes a reaction (oxygenase reaction) for fixing oxygen (O₂). As a rbcL gene derived from chloroplasts in the present invention, the rbcL gene derived from tobacco chloroplasts can be used preferably.

The accD gene constituting an expression cassette which enhances photosynthesis activity is a gene of acetyl CoA carboxylase encoded in the chloroplast genome. Acetyl CoA carboxylase is an enzyme involved in fatty acid synthesis in plants. As an accD gene derived from chloroplasts in the present invention, the accD gene derived from tobacco chloroplasts can be used preferably.

By using an expression cassette having a chloroplast-derived rbcL gene and a chloroplast-derived accD gene, a gene encoding a protein having FBPase and/or SBPase activities is easily integrated into a chloroplast by homologous recombination, and there is an advantage that an amount of expression of a protein having FBPase and/or SBPase activities is increased in chloroplasts.

In addition, it is not necessary to use the full length rbcL gene and accD gene. For example, those genes may be used as long as they have a sequence having a length of a base pair of about 1000 to 1500 on the rbcL gene side or accD gene side from a position into which a gene to be introduced is introduced, in a non-coding region between the rbcL gene and the accD gene, and being capable of homologous recombination with the rbcL gene or the accD gene.

In addition, the expression cassette for enhancing photosynthesis activity has a ribosome-binding site upstream of the translation initiation point of a DNA fragment which contains a gene encoding a protein having FBPase and/or SBPase activities. By placing the ribosome-binding site upstream of the DNA fragment, the protein can be highly expressed. The ribosome-binding site may be situated adjacent to and upstream of a translation initiation point of the gene encoding the protein, and it is preferably located about 7 to 11 bases upstream of the translation initiation point, further preferably about 9 bases upstream of the translation initiation point. Such ribosome-binding site is any nucleotide sequence as long as it has the known per se nucleotide sequence to which ribosomes can bind, and the SD sequence is preferable. The SD sequence is an abbreviation of the Shine-Dalgarno sequence, which is a segment consisting of 4 to 7 nucleotides, and its nucleotide sequence is a part or all of 5'-AGGAGGU-3' (SEQ ID NO: 18).

The expression cassette for enhancing photosynthesis activity further has a plant cell-derived promoter upstream of the ribosome-binding site. The promoter may be adjacent to a ribosome-binding site, or may be situated about 1 to 30 bases upstream, as long as it is located upstream of the ribosome-binding site. The promoter is the tobacco chloroplast-derived promoter psbA. Particularly, the tobacco chloroplast-derived psbA promoter described, for example, in SEQ ID NO: 7 of Sequence Listing can be used preferably.

The expression cassette for enhancing photosynthesis activity has a plant-derived terminator between a DNA fragment comprising a gene encoding a protein having FBPase and/or SBPase activities and the accD gene. The terminator may be adjacent to the DNA fragment, or may be situated about 1 to 30 bases downstream, as long as it is situated downstream of the DNA fragment. The terminator is a rps16 terminator. Inter alia, a chloroplast-derived terminator is preferable, a tobacco chloroplast-derived terminator is more preferable, the tobacco chloroplast-derived rps16 terminator is most preferable, and the tobacco chloroplast-derived rpa16 terminator described, for example, in SEQ ID NO: 8 of Sequence Listing can be used preferably.

In addition, it is preferable that an expression cassette has a gene for screening transformants. The gene for screening transformants is not particularly limited, and the known per se gene may be used. Examples of such gene include various drug resistance genes (aadA), and a gene compensating for auxotrophy of a host. More specific examples include an ampicillin resistance gene, a neomycin resistance gene (G418 resistant), a chloramphenicol resistance gene, a kanamycin resistance gene, a spectinomycin resistance gene, a URA3 gene and the like. More specifically, for example, a spectinomycin resistance gene described in SEQ IN NO: 9 of Sequence Listing can be used preferable. In addition, it is preferable that a promoter for recognizing the gene (hereinafter, abbreviated as aadA promoter) and a terminator of the gene (hereinafter, abbreviated as aadA terminator) are disposed upstream and downstream of the gene, respectively. As the aadA promoter and aadA terminator, the aforementioned plant-derived promoter and terminator can be preferably used, and the rrn promoter and the psbA terminator are particularly preferable. An aadA promoter/aadA/aadA terminator is referred to as aadA cassette in some cases.

It is preferable that an aadA cassette for screening transformants is disposed between a rbcL gene and a promoter upstream of the ribosome-binding site.

It is preferable that an expression cassette used in the vector of the present invention is constructed in an order of the rbcL gene, the aadA cassette, the promoter, the ribosome-binding site, the DNA fragment comprising a gene encoding a protein having FBPase and/or SBPase activities, the terminator, and the accD gene from the 5' side. Respective DNAs may be consecutive, or an intron sequence, for example, may be inserted between respective DNAs.

A recombinant gene vector of the present invention can be prepared, for example, by the following steps.

A first step is a step of making a pLD6 vector. Such vector can be easily made by the method described in Example [step 1]. A total nucleotide sequence of pLD6 is shown in SEQ ID NO: 10. In pLD6, a construction gene group is inserted into the cleavage site created by the NotI and SalI digestion of pLD6. The construction gene group has (a) a group consisting of a multicloning region (located from 3698 to 3748 in SEQ ID NO: 10) having a nucleotide sequence represented by SEQ ID NO: 11, the tobacco chloroplast-derivedpsbA promoter (located from 3569 to 3701 in SEQ ID NO: 10) represented by SEQ ID NO: 7 upstream therefrom, and the tobacco chloroplast-derived rps16 terminator (located from 3755 to 3913 in SEQ ID NO: 10) represented by SEQ ID NO: 8 downstream of the multicloning region, and, upstream of the group, (b) aadA gene (located from 2369 to 3173 in SEQ ID NO: 10) which is the spectinomycin resistance gene represented by SEQ ID NO: 9 as the gene for screen transformants, the tobacco chloroplast-derived rrn promoter (located from 2227 to 2368 in SEQ ID NO: 10) represented by SEQ ID NO: 12 upstream of the aadA gene, and a tobacco chloroplast-derived psbA terminator (located from 3175 to 3564 in SEQ ID NO: 10) represented by SEQ ID NO: 13 downstream of the aadA gene. The gene encoding a protein having FBPase and/or SBPase activities is inserted between restriction enzyme recognition sites (BglII, SphI, ClaI and EcoRI) of the aforementioned multicloning region. More specifically, for example, the gene encoding a spinach-derived FBPase represented by SEQ ID NO: 2 or the gene encoding spinach-derived SBPase represented by SEQ ID NO: 4, or the gene encoding a cyanobacterium-derived FBP/SBPase represented by SEQ In NO: 6 is inserted into the cleavage site created by the SphI and EcoRI digestion of the multicloning region of the pLD6 vector. In this case, the nucleotide sequence at 13 to 17 positions of SEQ ID NO: (5'-aggag-3') corresponds to the SD sequence, and functions as the ribosome-binding site. Hereinafter, a construction gene group in which the gene encoding a protein having FBPase and/or SBPase activities is inserted is referred to as FBP/SBP gene group, and the pLD6 vector in which the gene group is inserted is referred to as pLD6-FBP/SBP.

Then, pLD6-FBP/SBP is introduced into an appropriate host cell, and such host cell is cultured for cloning a FBP/SBP gene group.

A host cell can be appropriately selected from the known per se host cells, and examples thereof include prokaryotic organism such as Escherichia and Bacillus, eukaryotic organism such as yeast and filamentous fungus, plant cell or animal cell and the like. Condition for culturing a host cell may be' according to the condition which is normally performed in the art, depending on a kind of the host cell. In addition, whether the gene encoding a protein having FBPase and/or SBPase activities has been successfully introduced into a cloned gene or not can be easily determined based on a selective marker, etc. possessed by pLD6-FBP/SBP and the like.

The next step is making the pLD200 vector. Such vector can be easily prepared by the method described in Example [step 2]. A FBP/SBP gene group is excised, using NotI and SalI, from a recombinant gene which has been cloned using pLD6-FBP/SBP in the previous step, and the excised gene group is inserted between cleavage sites of NotI and SalI of the polylinker of pLD200. A total nucleotide sequence of pLD200 is described in SEQ ID NO: 14. The polylinker has a nucleotide sequence (located from 2125 to 2145 of SEQ ID NO: 14) represented by SEQ ID NO: 17, and has a plurality of restriction enzyme sites (NotI, NheI and SalI). The pLD200 vector is a vector characterized in that it has an expression cassette comprising the polylinker, the tobacco chloroplast-derived rbcL gene (located from 423 to 1856 in SEQ ID NO: 14) having a nucleotide sequence represented by SEQ ID NO: 15 upstream therefrom, and the tobacco chloroplast-derived accD gene (located from 2624 to 3328 in SEQ ID NO: 14) having a nucleotide sequence represented by SEQ ID NO: 16 downstream therefrom. Thus, the pLD200 vector in which a FBP/SBP gene group is inserted is referred to as pLD200-FBP/SBP.

The aforementioned vector may be also obtained by inserting a polylinker (preferably, a gene having a nucleotide sequence represented by SEQ ID NO: 17) having a plurality of restriction enzyme sites, the tobacco chloroplast-derived rbcL gene upstream of the polylinker, and the tobacco chloroplast-derived accD gene downstream of the polylinker into to the known per se cloning vectors.

The thus prepared pLD200-FBP/SBP is introduced into a host cell to prepare a transformant. Thereupon, as a host cell, a plant cell is preferable, chloroplasts are more preferable, and tobacco chloroplasts are further more preferable. In this way, by using a plant cell, particularly chloroplasts, as a host cell, there is an advantage that the protein encoded by the introduced gene can be highly expressed, and flying of the introduced gene into environment via pollen can be prevented.

As a method of introducing pLD200-FBP/SBP into a host cell, particularly, chloroplasts to perform transformation, the known methods may be used. Examples of such methods include a particle gun method in which the expression vector is dusted with extremely fine particles of gold or tungsten, and the particles to which the expression vector are adhered are shot into a host cell with a gunpowder or a high pressure gas to introduce the expression vector. Inter alia, it is preferable to use a procedure by a particle gun (Svab,Z., Hajdukiewicz,P., and Maliga,P., Proc.Natl.Acad.Scl.USA, 1990, vol.87, p.8526-8530), or a procedure by PEG (Golds, T., Maliga,P., and Koop,H.-U.,Bio/Technol., 1993, vol.11, p.95-97), in a system of introducing a gene into higher plant chloroplasts.

A plant having the aforementioned transformed chloroplasts of the present invention can be obtained by the known per se methods. Herein, the plant is not particularly limited, but higher plants are preferable, and a plant of which chloroplast transformation system is established is more preferable, including, for example, tobacco, rice, potato, rape and lettuce, and a tobacco is especially preferable. Examples of tobacco include *Nicotiana acuminate, Nicotiana alata, Nicotiana* attenuata, *Nicotiana clevelandii, Nicotiana excelsior, Nicotiana forgetiana, Nicotiana gossei, Nicotiana glauca, Nicotiana glutinosa, Nicotiana langsdorffii, Nicotiana longiflora, Nicotiana obtusifolia, Nicotiana paniculata, Nicotiana plumbagifolia, Nicotiana quadrivalvis, Nicotiana repanda, Nicotiana rustica, Nicotiana* sanderae, *Nicotiana suaveolens, Nicotiana sylvestris, Nicotiana tabacum, Nicotiana tomentosa, Nicotiana tomentosiformis and* the *like. Inter alia, Nicotiana rustica* and *Nicotiana tabacum* are preferable. In particular, *Nicotiana tabacum* is preferable and, among *Nicotiana* tabacum, "Burley", "Yellow (Virginia)", "Native" and "Oriental" are particularly preferable.

The aforementioned plants can be grown under the known per se condition depending on the plant.

Procedures of the genetic engineering or biotechnology can be easily performed by the methods described in commercially available experimental documents, for example, Molecular Cloning, Cold Spring Harbor Laboratory published in 1982, or Molecular Cloning, 2nd ed., Cold Spring Harbor Laboratory published in 1989, etc.

Vectors pLD6 andpLD200 utilized in a process of constructing a vector for introducing a gene into the tobacco chloroplast genome of the present invention are published in Japanese Patent Application No. 2001-083569.

The present invention will be explained in more detail by way of specific Example described below, but the present invention is not particularly limited thereto.

The meanings of respective abbreviations used in Example are as follows:
S.7942: Synechococcus PCC 7942
LB medium: Luria-Bertani medium
NaCl: sodium chloride

### Example

### Preparation of recombinant gene

### [Step 1] Preparation of pLD6-S.7942FBP/SBPase

A S.7942FBP/SBPase gene (fbp/sbp) represented by SEQ ID NO: 6 of Sequence Listing was inserted between restriction enzymes SphI and EcoRI sites of a vector pLD6 having the psbA promoter (PpsbA) by which high expression can be expected in tobacco chloroplasts, to prepare pLD6-S.7942FBP/SBPase. This pLD6-S.7942FBP/SBPase was introduced into Escherichia coli according to a conventional method. This Escherichia coli was cultured at 37°C for 16 hours in LB medium supplemented with spectinomycin to select the Escherichia coli in which such gene was introduced. The selected Escherichia coli was cultured under the similar condition, cells were collected by centrifugation, and pLD6-S.7942FBP/SBPase (plasmid DNA) was purified by a conventional method. The LB medium includes 10 g of tryptone, 5 g of yeast extract, and 5 g of NaCl per liter.

### [Step 2] Preparation of pLD200-S.7942FBP/SBPase

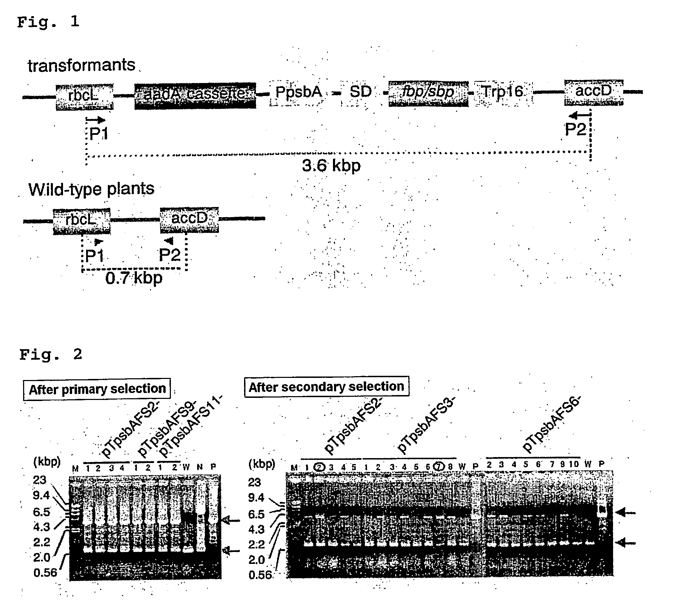

The pLD6-S.7942FBP/SBPase purified in the step 1 was treated with restriction enzymes NotI and SalI, and then the fragment containing S.7942FBP/SBPase was inserted between NotI and SalI sites of the vector pLD200 for transforming chloroplasts which' contains a part of the rbcL gene and a part of the accD gene of the tobacco chloroplast genome upstream of NotI and downstream of SalI, to prepare pLD200-S.7942FBP/SBPase. This pLD200-S.7942FBP/SBPase was introduced into Escherichia coli according to a conventional method. This Escherichia coli was cultured at 37°C for 16 hours in LB medium supplemented with spectinomycin to select the Escherichia coli in which such gene was introduced. The selected Escherichia coli was cultured under the similar condition, cells were collected by centrifugation, and pLD200-S.7942FBP/SBPase (plasmid DNA) was purified according to a conventional method (Fig. 1).

### [Step 3] Preparation of chloroplast transformant

The purified pLD200-S.7942FBP/SBPase was introduced into tobacco chloroplasts with a particle gun to prepare a chloroplast transformant. The transformation of tobacco chloroplasts was carried out according to the known method (Svab,Z., Hajdukiewicz,P. and maliga,P., Stable transformation of plastids in higher plants. Proc.Natl.Acad.Sci.USA, 87, 8526-8530(1990)).

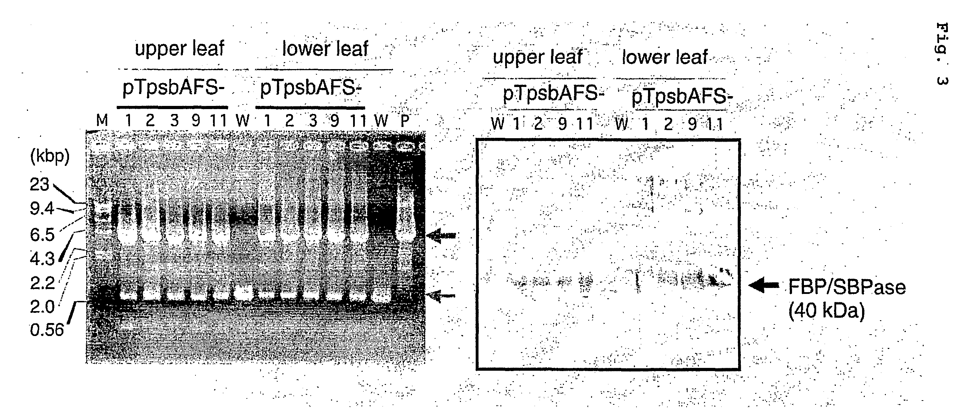

After redifferentiation on a spectinomycin-supplemented medium, a transformant (pTpsbAFS) 6 strain wherein S.7942FBP/SBPase was introduced into the chloroplast genome could be obtained by PCR. Also in T₁ generation produced by self hybridization, defect of the gene was not recognized (Fig. 2). Western blotting was performed using an anti-S.7942FBP/SBPase antibody and, as a result, the signal was recognized at a position of about 40kDa coinciding with a molecular mass of S. 7942FBP/SBPase only in the transformed plant (pTpsbAFS), and it was made clear that FBP/SBPase was highly expressed (Fig. 3).

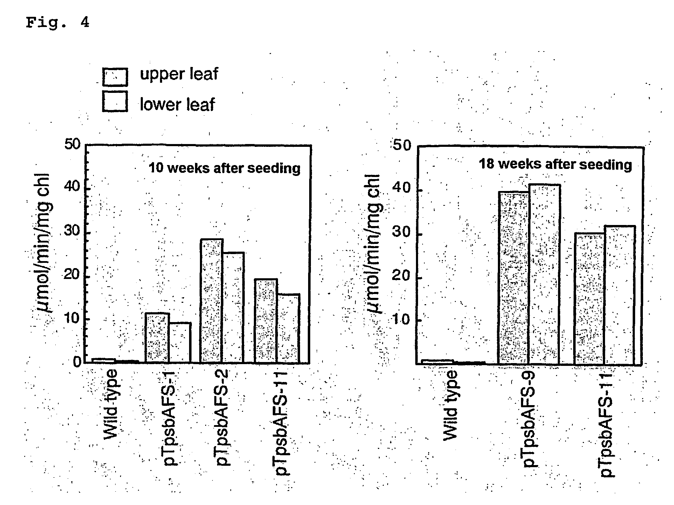

Using plants of 10 weeks and 18 weeks after seeding, FBPase activity was measured. The transformed plant had about 10 to 40-fold higher FBPase activity as compared with the wild strain (Fig. 4).

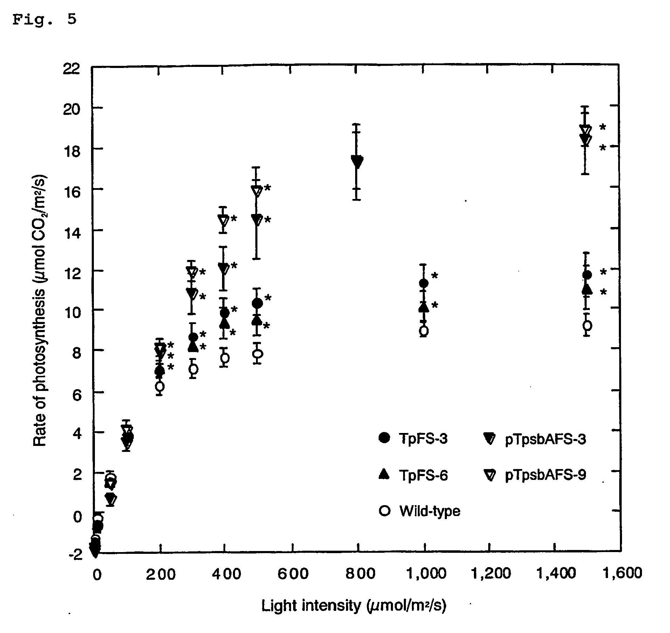

Using a T₁ generation 12 weeks after seeding, photosynthesis activity was measured by a change in light intensity under condition of the CO₂ concentration of 360 ppm. Results are shown in Fig. 5. Transformants (pTpsbAFS-3 and pTpsbAFS-9) and the wild strain (Wild-type) had a maximum photosynthesis rate at light intensity of about 500 µmol/m²/s and, thereafter, the rate was maintained. The photosynthesis rate of the transformant at a maximum was about 2-fold that of the wild strain.

For comparison, according to the method described in JP-A No. 2000-253768, the plasmid linked to S.7942FBP/SBPase was introduced into Agrobacterium tumefaciens LBA4404 to make a transformant (TpFS-3 and TPFS-6) infected with a leaf disk of tobacco. The TpFS-3 and TPFS-6 had an about 1.2 to 1.3-fold photosynthesis rate at a maximum as compared with a wild strain, which was far lower than the photosynthesis rates of pTpsbAFS-3 and pTpsbAFS-9. This demonstrates that the transformant of the present invention has an enhanced photosynthesis activity as compared with the wild strain and the transformed plant obtained by the conventional methods.

Furthermore, pTpsbAFS-3 and pTpsbAFS-9 showed a photosynthesis rate equivalent to a maximum of the wild strain at light intensity of about 200 µmol/m²/s, and a photosynthesis rate equivalent to a maximum of TpFS-3 and TPFS-6 at 300 µmol/m²/s. This demonstrates that the transformed plant of the present invention has sufficient photosynthesis activity even when light intensity is low.

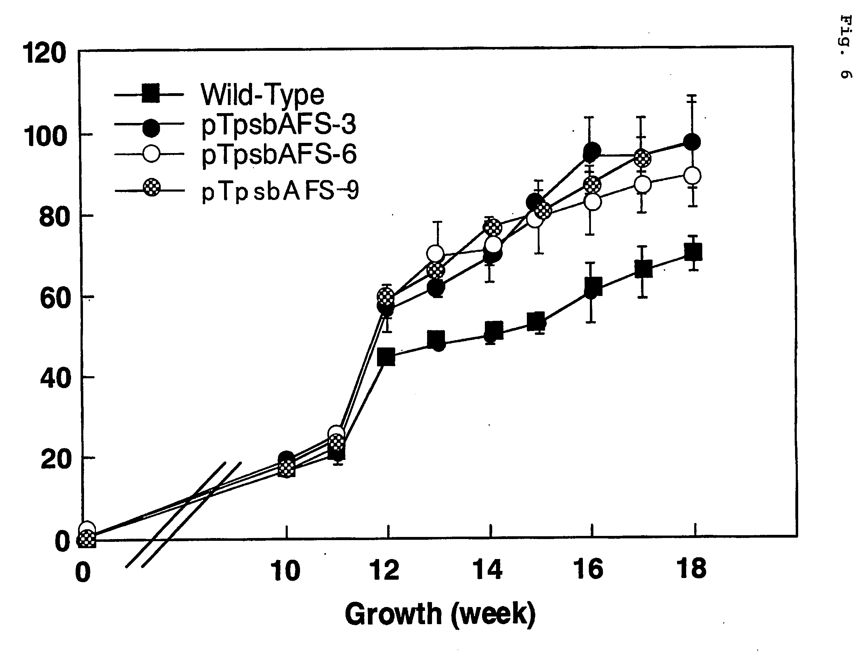

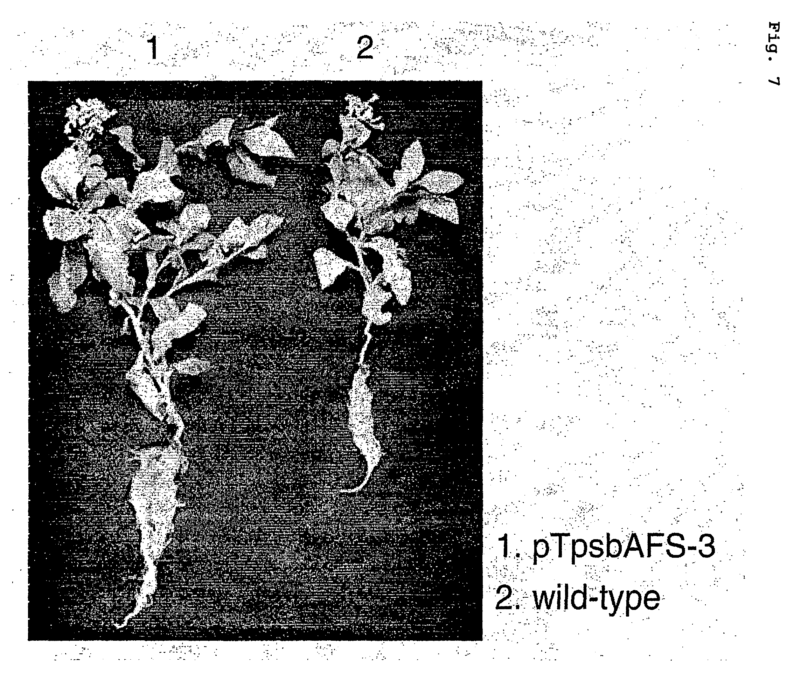

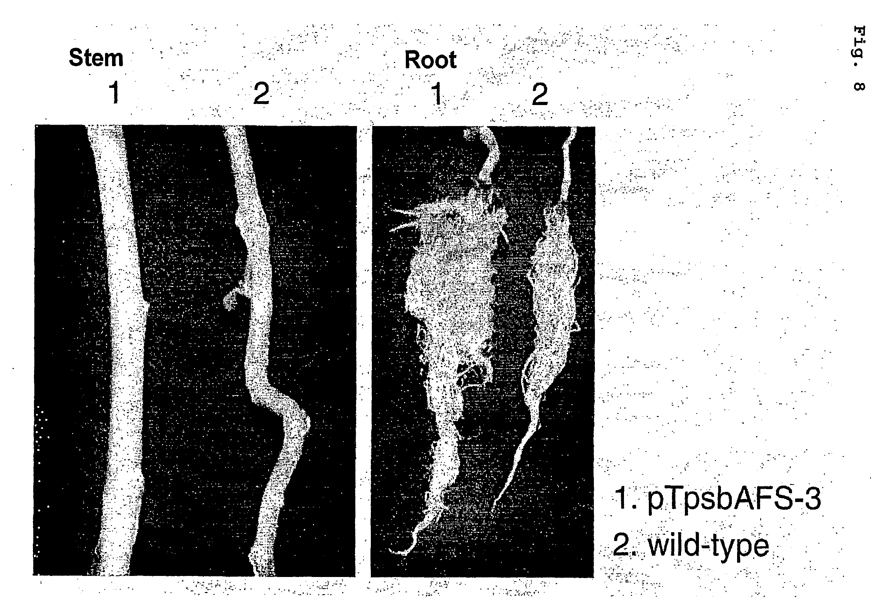

When growth of the transformants and growth of the wild strain were compared 18 weeks after seeding, growth of the transformed plants was clearly promoted as compared with the wild strain, and the final growth reached 1.2 to 1.3-fold that of a wild strain (Fig. 6 and 7). In addition, a stem of a transformant was thicker than that of a wild strain, and also a root was remarkably developed (Fig. 8). Further, after 18 weeks, transformants had grown to be about 1.5-fold the size of a wild strain.

As mentioned above, by introducing a S.7942FBP/SBPase gene into the tobacco chloroplast genome, photosynthesis ability of tobacco leaves could be enhanced. Further, thereby, it becomes possible to promote growth, and increase the yield.

With respect to plants other than tobacco, a plant cell into which the S.7942FBP/SBP gene can be introduced and expressed can be prepared by introducing the aforementioned plasmid pLD200-S.7942FBP/SBP into chloroplasts with a particle gun, and selecting a resistant cell in a medium supplemented with spectinomycin.

For example, a transformed plant cell can be prepared by discharging the aforementioned plasmid with a particle gun into rape seed leaf, potato leaf blade, lettuce leaf blade, rice leaf blade or embryonic stem cell, and selecting a resistant cell on a spectinomycin-supplemented medium with an appropriate concentration. The resultant cells in which the S.7942FBP/SBP gene is introduced and expressed are redifferentiated under appropriate conditions, thereby to produce a plant having' improved photosynthesis ability which promotes the growth. The transformation conditions for rape are described in Transgenic Research, 12(1), p.111-114 (2003), those for potato in Plant Journal, 19(2), p.209-216 (1999), those for rice in Nature Biotechnology, 17(9) p.910-915 (1999) and those for lettuce in Sympodium of Japanese Society for Plant Cell and Molecular Biology, 1Da-10, 2004.

Similarly, with respect to other plant species, a transformed plant can be produced by discharging the aforementioned pLD200-S.7942FBP/SBP gene into a leaf blade or an embryonic stem cell with a particle gun, selecting a resistant cell on the spectinomycin-supplemented medium and redifferentiating the selected cell. The selection conditions using spectinomycin can be easily determined by observation of the growth and redifferentiation in the medium supplemented with various concentrations of spectinomycin. Usually, it is preferred to select a condition wherein a wild type strain cannot grow at a concentration as low as possible. The condition for redifferentiation of a callus into a plant can be determined by the conventional technique. For example, selection is carried out using a matrix medium containing auxin or cytokinin with a stepwise varied concentration, and optimum conditions for redifferentiation are determined. If required, gibberellin or amino acids may be added in some cases. Redifferentiation conditions from a callus in a variety of plant species into plants have been determined today. When the aforementioned chloroplast transformation technique is applied to these plants, there can be obtained a plant wherein the S.7942FBP/SBP gene has been introduced and expressed and which has an improved' photosynthesis ability promoting the growth. With respect to plants redifferentiation conditions of which have not been established so far, when such redifferentiation becomes possible in the future, application of the vector of the present invention to such plants for introduction and expression of the S.7942FBP/SBP gene should make it possible to produce a plant whose growth is promoted due to its improved photosynthesis ability.

### Industrial Applicability

A plant transformed using the gene recombinant of the present invention has high photosynthesis activity, and is useful as a quickly growing plant or a high yield plant.

## Claims

1. A recombinant gene vector comprising an expression cassette containing a DNA fragment comprising a gene encoding a protein having FBPase and/or SBPase activities between tobacco-derived Rubisco large subunit rbcL gene and tobacco-derived acetyl CoA carboxylase subunit accD gene, having a ribosome-binding site upstream of a translation initiation point of the DNA fragment, having tobacco-derived psbA promoter between the Rubisco large subunit gene and the ribosome-binding site, and having a tobacco-derived rps16 terminator between the acetyl CoA carboxylase subunit gene and the DNA fragment.

2. A transformed chloroplast **characterized in that** the vector according to claim 1 is introduced into chloroplasts.

3. A plant containing transformed chloroplasts according to claim 2.

4. The plant as claimed in claim 3, wherein the plant is tobacco.

5. The recombinant gene vector as claimed in claim 1, wherein the gene encoding a protein having FBPase and/or SBPase activities is derived from Spinach.

6. The recombinant gene vector as claimed in claim 5, wherein the Spinach-derived gene consists of the DNA sequence set forth in SEQ ID NO:2 or SEQ ID NO:4.

7. The recombinant gene vector as claimed in claim 1, wherein the gene encoding a protein having FBPase and/or SBPase activities is derived from Synechococcus.

8. The recombinant gene vector as claimed in claim 7, wherein the Synechococcus-derived gene consists of the DNA sequence set forth in SEQ ID NO:6.

9. The recombinant gene vector as claimed in any one of claims 1, 5, 6, 7 and 8, wherein the rbcL gene consists of the DNA sequence set forth in SEQ ID NO:15, the accD gene consists of the DNA sequence set forth in SEQ ID NO:16, the psbA promoter consists of the DNA sequence set forth in SEQ ID NO:7, and the rps16 terminator consists of the DNA sequence set forth in SEQ ID NO:8.

## Patentansprüche

1. Rekombinanter Genvektor, umfassend eine Expressionskassette, die ein DNA-Fragment, umfassend ein Gen, das ein Protein mit FBPase- und/oder SBPase-Aktivität kodiert, zwischen dem rbcL-Gen der großen Untereinheit des von Tabak stammenden Rubiscos und dem accD-Gen der Untereinheit der von Tabak stammenden Acetyl-CoA-Carboxylase enthält, eine Ribosom-Bindungsstelle stromaufwärts eines Translationsinitiationspunktes des DNA-Fragments aufweist, einen von Tabak stammenden psbA-Promotor zwischen dem Gen der großen Untereinheit des Rubiscos und der Ribosom-Bindungsstelle aufweist und einen von Tabak stammenden rps-16-Terminator zwischen dem Gen der Untereinheit der Acetyl-CoA-Carboxylase und dem DNA-Fragment aufweist.

2. Transformierter Chloroplast, **dadurch gekennzeichnet, daß** der Vektor nach Anspruch 1 in Chloroplasten eingeführt ist.

3. Pflanze, die transformierte Chloroplasten nach Anspruch 2 enthält.

4. Pflanze nach Anspruch 3, wobei die Pflanze Tabak ist.

5. Rekombinanter Genvektor nach Anspruch 1, wobei das Gen, das ein Protein mit FBPase-und/oder SBPase-Aktivität kodiert, von Spinat stammt.

6. Rekombinanter Genvektor nach Anspruch 5, wobei das von Spinat stammende Gen aus der DNA-Sequenz besteht, die in SEQ ID Nr.: 2 oder SEQ ID Nr.: 4 dargestellt ist.

7. Rekombinanter Genvektor nach Anspruch 1, wobei das Gen, das ein Protein mit FBPase-und/oder SBPase-Aktivität kodiert, von Synechococcus stammt.

8. Rekombinanter Genvektor nach Anspruch 7, wobei das von Synechococcus stammende Gen aus der DNA-Sequenz besteht, die in SEQ ID Nr.: 6 dargestellt ist.

9. Rekombinanter Genvektor nach einem der Ansprüche 1, 5, 6, 7 und 8, wobei das rbcL-Gen aus der DNA-Sequenz besteht, die in SEQ ID Nr.: 15 dargestellt ist, das accD-Gen aus der DNA-Sequenz besteht, die in SEQ ID Nr.: 16 dargestellt ist, der psbA-Promotor aus der DNA-Sequenz besteht, die in SEQ ID Nr.: 7 dargestellt ist und der rps-16-Terminator aus der DNA-Sequenz besteht, die in SEQ ID Nr.: 8 dargestellt ist.

## Revendications

1. Vecteur de gène recombinant comprenant une cassette d'expression contenant un fragment d'ADN comprenant un gène codant pour une protéine ayant des activités de FBPase et/ou SBPase entre le gène rbcL de grande sous-unité de la RubisCO dérivée du tabac et le gène accD de sous-unité d'acétyl CoA carboxylase dérivée du tabac, comportant un site de liaison ribosomique en amont d'un point d'initiation de traduction du fragment d'ADN, comportant un promoteur psbA dérivé du tabac entre le gène de grande sous-unité de la RubisCO et le site de liaison ribosomique, et comportant un terminateur rps16 dérivé du tabac entre le gène de sous-unité d'acétyl CoA carboxylase et le fragment d'ADN.

2. Chloroplaste transformé, **caractérisé en ce que** le vecteur suivant la revendication 1 est introduit dans des chloroplastes.

3. Plante contenant des chloroplastes transformés suivant la revendication 2.

4. Plante suivant la revendication 3, dans laquelle la plante est du tabac.

5. Vecteur de gène recombinant suivant la revendication 1, dans lequel le gène codant pour une protéine ayant des activités de FBPase et/ou SBPase est dérivé de l'épinard.

6. Vecteur de gène recombinant suivant la revendication 5, dans lequel le gène dérivé de l'épinard est formé de la séquence d'ADN formulée dans SEQ ID NO :2 ou SEQ ID NO :4.

7. Vecteur de gène recombinant suivant la revendication 1, dans lequel le gène codant pour une protéine ayant des activités de FBPase et/ou SBPase est dérivé de Synechococcus.

8. Vecteur de gène recombinant suivant la revendication 7, dans lequel le gène dérivé de Synechococcus est formé de la séquence d'ADN formulée dans SEQ ID NO :6.

9. Vecteur de gène recombinant suivant l'une quelconque des revendications 1, 5, 6, 7 et 8, dans lequel le gène rbcL est formé de la séquence d'ADN formulée dans SEQ ID NO :15, le gène accD est formé de la séquence d'ADN formulée dans SEQ IDNO :16, le promoteur psbA est formé de la séquence d'ADN formulée dans SEQ ID NO :7, et le terminateur rps16 est formé de la séquence d'ADN formulée dans SEQ ID NO :8.


### SEQUENCE LISTING

<110> National University corporation Nara Institute of science and Technology,
   Research Institute of Innovative Technology for the Earth, Kinki university
<120> Transgenic plants
<130> C01F1576
<160> 18
<210> 1
   <211> 358
   <212> PRT
   <213> Spinacia oleracea L
<220>
   <223> Fructose-1,6-bisphosphatase
<400> 1
   Ala Ala Val Gly Glu Ala Ala Thr Glu Thr Lys Ala Arg Thr Arg Ser
                   5                   10                  15
   Lys Tyr Glu Ile Glu Thr Leu Thr Gly Trp Leu Leu Lys Gln Glu Met
               20                  25                  30
   Ala Gly Val Ile Asp Ala Glu Leu Thr Ile Val Leu Ser Ser Ile Ser
           35                  40                  45
   Leu Ala Cys Lys Gln Ile Ala Ser Leu Val Gln Arg Ala Gly Ile Ser
       50                  55                  60
   Asn Leu Thr Gly Ile Gln Gly Ala Val Asn Ile Gln Gly Glu Asp Gln
   65                  70                  75                  80
   Lys Lys Leu Asp Val Val Ser Asn Glu Val Phe Ser Ser Cys Leu Arg
                   85                  90                  95
   Ser Ser Gly Arg Thr Gly Ile Ile Ala Ser Glu Glu Glu Asp Val Pro
               100                 105                 110
   Val Ala Val Glu Glu Ser Tyr Ser Gly Asn Tyr Ile Val Val Phe Asp
           115                 120                 125
   Pro Leu Asp Gly Ser Ser Asn Ile Asp Ala Ala Val Ser Thr Gly Ser
       130                 135                 140
   Ile Phe Gly Ile Tyr Ser Pro Asn Asp Glu Cys Ile Val Asp Ser Asp
   145                 150                 155                 160
   His Asp Asp Glu Ser Gln Leu Ser Ala Glu Glu Gln Arg Cys Val Val
                   165                 170                 175
   Asn Val Cys Gln Pro Gly Asp Asn Leu Leu Ala Ala Gly Tyr Cys Met
               180                 185                 190
   Tyr Ser Ser Ser Val Ile Phe Val Leu Thr Ile Gly Lys Gly Val Tyr
           195                 200                 205
   Ala Phe Thr Leu Asp Pro Met Tyr Gly Glu Phe Val Leu Thr Ser Glu
       210                 215                 220
   Lys Ile Gln Ile Pro Lys Ala Gly Lys Ile Tyr Ser Phe Asn Glu Gly
   225                 230                 235                 240
   Asn Tyr Lys Met Trp Asp Asp Lys Leu Lys Lys Tyr Met Asp Asp Leu
                   245                 250                 255
   Lys Glu Pro Gly Glu Ser Gln Lys Pro Tyr Ser Ser Arg Tyr Ile Gly
               260                 265                 270
   Ser Leu Val Gly Asp Phe His Arg Thr Leu Leu Tyr Gly Gly Ile Tyr
           275                 280                 285
   Gly Tyr Pro Arg Asp Ala Lys Ser Lys Asn Gly Lys Leu Arg Leu Leu
       290                 295                 300
   Tyr Glu Cys Ala Pro Met Ser Phe Ile Val Glu Gln Ala Gly Gly Lys
   305                 310                 315                 320
   Gly Ser Asp Gly His Gln Arg Ile Leu Asp Ile Gln Pro Thr Glu Ile
                   325                 330                 335
   His Gln Arg Val Pro Leu Tyr Ile Gly Ser Val Glu Glu Val Glu Lys
               340                 345                 350
   Leu Glu Lys Tyr Leu Ala
           355
<210> 2
   <211> 1074
   <212> DNA
   <213> spinacia oleracea L
<220>
   <223> Fructose-1,6-bisphosphatase
<400> 2
   gcagccgtag gagaggcggc tacagaaaca aaggcaagga ctagaagtaa gtacgaaatt 60
   gaaacactaa caggctggct gcttaaacaa gaaatggcag gtgttattga tgctgaactt 120
   accatcgttc tttctagcat ttcattggct tgtaaacaaa ttgcttcctt ggttcaacga 180
   gctggtattt ctaacttgac tggaattcaa ggtgctgtca atatccaagg agaggatcag 240
   aagaaacttg atgttgtctc caatgaggtg ttttcgagct gcttgagatc gagtggaaga 300
   acaggaataa tagcatcaga agaagaggat gtaccagtgg cagtggaaga gagttactct 360
   ggaaactata ttgttgtgtt tgatccactt gatggttcat ccaacattga tgcagctgtc 420
   tccactggtt ccatctttgg catttatagc cctaacgatg agtgcattgt tgactctgat 480
   cacgacgatg agtcacagct aagtgcagaa gaacagaggt gtgtagtgaa tgtatgtcaa 540
   ccaggggata acctattagc agcagggtat tgtatgtact caagctctgt tatcttcgta 600
   cttacaattg gtaaaggtgt gtatgcattc acattagatc caatgtatgg tgaattcgta 660
   ctcacttcag agaaaatcca aatcccaaaa gctgggaaga tctattcatt caatgaaggt 720
   aactacaaaa tgtgggatga taaattgaag aagtacatgg atgatcttaa agagccagga 780
   gagtcacaga aaccgtactc gtctcgttac atagggagtt tagttgggga ctttcataga 840
   acacttttat atggtgggat ttatggttac ccaagagatg caaagagtaa gaatgggaaa 900
   ttgaggcttt tgtatgaatg tgcacctatg agttttattg ttgaacaagc tggtggtaaa 960
   ggttctgatg gtcatcaaag aattcttgac attcaaccca ccgagataca tcaacgtgtg 1020
   ccactgtaca tcgggagtgt ggaggaagta gagaaattag agaagtactt agca 1074
<210> 3
   <211> 333
   <212> PRT
   <213> Spinacia oleracea L
<220>
   <223> Sedoheptulose-1,7-bisphosphatase
<400> 3
   Val Asn Lys Ala Lys Asn Ser Ser Leu Val Thr Lys Cys Glu Leu Gly
                   5                   10                  15
   Asp Ser Leu Glu Glu Phe Leu Ala Lys Ala Thr Thr Asp Lys Gly Leu
               20                  25                  30
   Ile Arg Leu Met Met Cys Met Gly Glu Ala Leu Arg Thr Ile Gly Phe
           35                  40                  45
   Lys Val Arg Thr Ala Ser Cys Gly Gly Thr Gln Cys Val Asn Thr Phe
       50                  55                  60
   Gly Asp Glu Gln Leu Ala Ile Asp Val Leu Ala Asp Lys Leu Leu Phe
   65                  70                  75                  80
   Glu Ala Leu Asn Tyr Ser His Phe Cys Lys Tyr Ala Cys Ser Glu Glu
                   85                  90                  95
   Leu Pro Glu Leu Gln Asp Met Gly Gly Pro Val Asp Gly Gly Phe Ser
               100                 105                 110
   Val Ala Phe Asp Pro Leu Asp Gly Ser Ser Ile Val Asp Thr Asn Phe
           115                 120                 125
   Ser Val Gly Thr Ile Phe Gly Val Trp Pro Gly Asp Lys Leu Thr Gly
       130                 135                 140
   Val Thr Gly Arg Asp Gln Val Ala Ala Ala Met Gly Ile Tyr Gly Pro
   145                 150                 155                 160
   Arg Thr Thr Tyr Val Leu Ala Leu Lys Asp Tyr Pro Gly Thr His Glu
                   165                 170                 175
   Phe Leu Leu Leu Asp Glu Gly Lys Trp Gln His Val Lys Glu Thr Thr
               180                 185                 190
   Glu Ile Asn Glu Gly Lys Leu Phe Cys Pro Gly Asn Leu Arg Ala Thr
           195                 200                 205
   Ser Asp Asn Ala Asp Tyr Ala Lys Leu Ile Gln Tyr Tyr Ile Lys Glu
       210                 215                 220
   Lys Tyr Thr Leu Arg Tyr Thr Gly Gly Met Val Pro Asp Val Asn Gln
   225                 230                 235                 240
   Ile Ile Val Lys Glu Lys Gly Ile Phe Thr Asn Val Ile Ser Pro Thr
                   245                 250                 255
   Ala Lys Ala Lys Leu Arg Leu Leu Phe Glu Val Ala Pro Leu Gly Phe
               260                 265                 270
   Leu Ile Glu Lys Ala Gly Gly His Ser Ser Glu Gly Thr Lys Ser Val
           275                 280                 285
   Leu Asp Ile Glu Val Lys Asn Leu Asp Asp Arg Thr Gln Val Ala Tyr
       290                 295                 300
   Gly Ser Leu Asn Glu Ile Ile Arg Phe Glu Lys Thr Leu Tyr Gly Ser
   305                 310                 315                 320
   Ser Arg Leu Glu Glu Pro Val Pro Val Gly Ala Ala Ala
                   325                 330
<210> 4
   <211> 999
   <212> DNA
   <213> Spinacia oleracea L
<220>
   <223> Sedoheptulose-1,7-bisphosphatase
<400> 4
   gtgaacaagg caaagaactc ttcccttgta accaaatgtg aacttggtga cagtttggag      60
   gagttcctag caaaggcaac cacagataaa gggctgatta gattgatgat gtgcatggga     120
   gaagcattaa ggaccattgg ctttaaagtg aggactgctt catgtggtgg aactcaatgt     180
   gttaacacct ttggagacga acagcttgcc attgatgtgc ttgctgacaa gcttcttttc     240
   gaggcattga actattcaca cttctgcaag tatgcttgtt cagaagaact ccctgagctt     300
   caagatatgg gaggccccgt tgatggcgga ttcagtgtag catttgaccc ccttgatgga     360
   tccagcattg tcgataccaa tttctcagtt gggaccatat tcggggtttg gccaggtgac     420
   aagctaactg gtgtaacagg cagagatcaa gtggctgctg caatgggaat ttatggtcct     480
   aggactactt atgttctcgc tcttaaggac taccctggca cccatgaatt tcttcttctt     540
   gatgaaggaa agtggcaaca tgtgaaagaa acaacagaaa tcaatgaagg aaaattgttc     600
   tgtcctggaa acttgagagc cacttctgac aatgctgatt atgctaagct gattcaatac     660
   tatataaaag agaaatacac attgagatac actggaggaa tggttcctga tgttaaccag     720
   atcatagtga aggagaaagg tatattcaca aatgtaatat cacctacagc caaggcaaag     780
   ttgaggttac tgtttgaggt agctcctcta gggttcttga ttgagaaggc tggtggtcac     840
   agcagtgagg gaaccaagtc tgtgttggac attgaagtca aaaaccttga tgacagaacc     900
   caagttgctt acggctcctt gaacgagatc atccgatttg agaagacact atacggatcc     960
   tctaggctag aggagccagt tcctgttgga gctgctgct                            999
<210> 5
   <211> 356
   <212> PRT
   <213> Synechococcus
<220>
   <223> fructose-1,6-bisphosphatase/sedoheptulose-1,7-bisphosphatase from Synechococcus PCC 7942
<400> 5
   Met Glu Lys Thr Ile Gly Leu Glu Ile Ile Glu Val Val Glu Gln Ala
                   5                   10                  15
   Ala Ile Ala Ser Ala Arg Leu Met Gly Lys Gly Glu Lys Asn Glu Ala
               20                  25                  30
   Asp Arg Val Ala Val Glu Ala Met Arg Val Arg Met Asn Gln Val Glu
           35                  40                  45
   Met Leu Gly Arg Ile Val Ile Gly Glu Gly Glu Arg Asp Glu Ala Pro
       50                  55                  60
   Met Leu Tyr Ile Gly Glu Glu Val Gly Ile Tyr Arg Asp Ala Asp Lys
   65                  70                  75                  80
   Arg Ala Gly Val Pro Ala Gly Lys Leu Val Glu Ile Asp Ile Ala Val
                   85                  90                  95
   Asp Pro Cys Glu Gly Thr Asn Leu Cys Ala Tyr Gly Gln Pro Gly Ser
               100                 105                 110
   Met Ala Val Leu Ala Ile Ser Glu Lys Gly Gly Leu Phe Ala Ala Pro
           115                 120                 125
   Asp Phe Tyr Met Lys Lys Leu Ala Ala Pro Pro Ala Ala Lys Gly Lys
       130                 135                 140
   Glu Thr Ser Ile Lys Ser Ala Thr Glu Asn Leu Lys Ile Leu Ser Glu
   145                 150                 155                 160
   Cys Leu Asp Arg Ala Ile Asp Glu Leu Val Val Val Val Met Asp Arg
                   165                 170                 175
   Pro Arg His Lys Glu Leu Ile Gln Glu Ile Arg Gln Ala Gly Ala Arg
               180                 185                 190
   Val Arg Leu Ile Ser Asp Gly Asp Val Ser Ala Ala Ile Ser Cys Gly
           195                 200                 205
   Phe Ala Gly Thr Asn Thr His Ala Leu Met Gly Ile Gly Ala Ala Pro
       210                 215                 220
   Glu Gly Val Ile Ser Ala Ala Ala Met Arg Cys Leu Gly Gly His Phe
   225                 230                 235                 240
   Gln Gly Gln Leu Ile Tyr Asp Pro Glu Val Val Lys Thr Gly Leu Ile
                   245                 250                 255
   Gly Glu Ser Arg Glu Ser Asn Ile Ala Arg Leu Gln Glu Met Gly Ile
               260                 265                 270
   Thr Asp Pro Asp Arg Val Tyr Asp Ala Asn Glu Leu Ala Ser Gly Gln
           275                 280                 285
   Glu Val Leu Phe Ala Ala Cys Gly Ile Thr Pro Gly Leu Leu Met Glu
       290                 295                 300
   Gly Val Arg Phe Phe Lys Gly Gly Ala Arg Thr Gln Ser Leu Val Ile
   305                 310                 315                 320
   Ser Ser Gln Ser Arg Thr Ala Arg Phe Val Asp Thr Val His Met Phe
                   325                 330                 335
   Asp Asp Val Lys Thr Val Ser Leu Pro Leu Ile Pro Asp Pro Lys Trp
               340                 345                 350
   Arg Pro Glu Arg
           355
<210> 6
   <211> 1312
   <212> DNA
   <213> Synechococcus
<220>
   <223> fructose-1,6-bisphosphatase/sedoheptulose-1,7-bisphosphatase from Synechococcus PCC 7942
<400> 6
   atcgcaacta aagccagaga tgtgaggagg ggatccggcc tttggtagac tcaactgttg      60
   gaatccccag aagcaatcat ccgtaaggag tcaggacggc gtggagaaga cgatcggtct     120
   cgagattatt gaagttgtcg agcaggcagc gatcgcctcg gcccgcctga tgggcaaagg     180
   cgaaaagaat gaagccgatc gcgtcgcagt agaagcgatg cgggtgcgga tgaaccaagt     240
   ggaaatgctg ggccgcatcg tcatcggtga aggcgagcgc gacgaagcac cgatgctcta     300
   tatcggtgaa gaagtgggca tctaccgcga tgcagacaag cgggctggcg taccggctgg     360
   caagctggtg gaaatcgaca tcgccgttga cccctgcgaa ggcaccaacc tctgcgccta     420
   cggtcagccc ggctcgatgg cagttttggc catctccgag aaaggcggcc tgtttgcagc     480
   tcccgacttc tacatgaaga aactggctgc acccccagct gccaaaggca aagagacatc     540
   aataaagtcc gcgaccgaaa acctgaaaat tctctcggaa tgtctcgatc gcgccatcga     600
   tgaattggtg gtcgtggtca tggatcgtcc ccgccacaaa gagctaatcc aagagatccg     660
   ccaagcgggt gcccgcgtcc gtctgatcag cgatggtgac gtttcggccg cgatctcctg     720
   cggttttgct ggcaccaaca cccacgccct gatgggcatc ggtgcagctc ccgagggtgt      780
   gatttcggca gcagcaatgc gttgcctcgg cgggcacttc caaggccagc tgatctacga     840
   cccagaagtg gtcaaaaccg gcctgatcgg tgaaagccgt gagagcaaca tcgctcgcct     900
   gcaagaaatg ggcatcaccg atcccgatcg tgtctacgac gcgaacgaac tggcttcggg     960
   tcaagaagtg ctgtttgcgg cttgcggtat caccccgggc ttgctgatgg aaggcgtgcg    1020
   cttcttcaaa ggcggcgctc gcacccagag cttggtgatc tccagccagt cacggacggc    1080
   tcgcttcgtt gacaccgttc acatgttcga cgatgtcaaa acggttagcc tgccgttaat    1140
   tcctgatccc aaatggcggc cggagcggta gaacgggtat agctcgatcg cttcggtcgt    1200
   tgtttttcag cgaatccatt tgcgatcgct tttcaaaccc ttttttcgtc aaccttcttt     1260
   aaacggcctc atgcatctcg cagttgtcgg ctcagccatc ggacagcacc gg            1312
<210> 7
   <211> 133
   <212> DNA
   <213> Nicotiana tabacum
<220>
   <223> psbA promoter
<400> 7
   agcttctaca tacaccttgg ttgacacgag tatataagtc atgttatact gttgaataac 60
   aagccttcca ttttctattt tgatttgtag aaaactagtg tgcttgggag tccctgatga 120
   ttaaataaac caa 133
<210> 8
   <211> 159
   <212> DNA
   <213> Nicotiana tabacum
<220>
   <223> rps16 terminator
<400> 8
   agcttgaaat tcaattaagg aaataaatta aggaaataca aaaagggggg tagtcatttg 60
   tatataactt tgtatgactt ttctcttcta tttttttgta tttcctccct ttccttttct 120
   atttgtattt ttttatcatt gcttccattg aattactag 159
<210> 9
   <211> 805
   <212> DNA
   <213> Escherichia coli
<220>
   <223> aadA
<400> 9
   gatccatggc tcgtgaagcg gttatcgccg aagtatcaac tcaactatca gaggtagttg 60
   gcgtcatcga gcgccatctc gaaccgacgt tgctggccgt acatttgtac ggctccgcag 120
   tggatggcgg cctgaagcca cacagtgata ttgatttgct ggttacggtg accgtaaggc 180
   ttgatgaaac aacgcggcga gctttgatca acgacctttt ggaaacttcg gcttcccctg 240
   gagagagcga gattctccgc gctgtagaag tcaccattgt tgtgcacgac gacatcattc 300
   cgtggcgtta tccagctaag cgcgaactgc aatttggaga atggcagcgc aatgacattc 360
   ttgcaggtat cttcgagcca gccacgatcg acattgatct ggctatcttg ctgacaaaag 420
   caagagaaca tagcgttgcc ttggtaggtc cagcggcgga ggaactcttt gatccggttc 480
   ctgaacagga tctatttgag gcgctaaatg aaaccttaac gctatggaac tcgccgcccg 540
   actgggctgg cgatgagcga aatgtagtgc ttacgttgtc ccgcatttgg tacagcgcag 600
   taaccggcaa aatcgcgccg aaggatgtcg ctgccgactg ggcaatggag cgcctgccgg 660
   cccagtatca gcccgtcata cttgaagcta gacaggctta tcttggacaa gaagaagatc 720
   gcttggcctc gcgcgcagat cagttggaag aatttgtcca ctacgtgaaa ggcgagatca 780
   ctaaggtagt tggcaaataa ctgca 805
<210> 10
   <211> 4591
   <212> DNA
   <213> Artificial sequence
<220>
   <223> pLD6
<400> 10
   gtggcacttt tcggggaaat gtgcgcggaa cccctatttg tttatttttc taaatacatt      60
   caaatatgta tccgctcatg agacaataac cctgataaat gcttcaataa tattgaaaaa     120
   ggaagagtat gagtattcaa catttccgtg tcgcccttat tccctttttt gcggcatttt     180
   gccttcctgt ttttgctcac ccagaaacgc tggtgaaagt aaaagatgct gaagatcagt     240
   tgggtgcacg agtgggttac atcgaactgg atctcaacag cggtaagatc cttgagagtt     300
   ttcgccccga agaacgtttt ccaatgatga gcacttttaa agttctgcta tgtggcgcgg     360
   tattatcccg tattgacgcc gggcaagagc aactcggtcg ccgcatacac tattctcaga     420
   atgacttggt tgagtactca ccagtcacag aaaagcatct tacggatggc atgacagtaa     480
   gagaattatg cagtgctgcc ataaccatga gtgataacac tgcggccaac ttacttctga     540
   caacgatcgg aggaccgaag gagctaaccg cttttttgca caacatgggg gatcatgtaa     600
   ctcgccttga tcgttgggaa ccggagctga atgaagccat accaaacgac gagcgtgaca     660
   ccacgatgcc tgtagcaatg gcaacaacgt tgcgcaaact attaactggc gaactactta     720
   ctctagcttc ccggcaacaa ttaatagact ggatggaggc ggataaagtt gcaggaccac     780
   ttctgcgctc ggcccttccg gctggctggt ttattgctga taaatctgga gccggtgagc     840
   gtgggtctcg cggtatcatt gcagcactgg ggccagatgg taagccctcc cgtatcgtag     900
   ttatctacac gacggggagt caggcaacta tggatgaacg aaatagacag atcgctgaga     960
   taggtgcctc actgattaag cattggtaac tgtcagacca agtttactca tatatacttt    1020
   agattgattt aaaacttcat ttttaattta aaaggatcta ggtgaagatc ctttttgata    1080
   atctcatgac caaaatccct taacgtgagt tttcgttcca ctgagcgtca gaccccgtag    1140
   aaaagatcaa aggatcttct tgagatcctt tttttctgcg cgtaatctgc tgcttgcaaa    1200
   caaaaaaacc accgctacca gcggtggttt gtttgccgga tcaagagcta ccaactcttt 1260
   ttccgaaggt aactggcttc agcagagcgc agataccaaa tactgtcctt ctagtgtagc 1320
   cgtagttagg ccaccacttc aagaactctg tagcaccgcc tacatacctc gctctgctaa 1380
   tcctgttacc agtggctgct gccagtggcg ataagtcgtg tcttaccggg ttggactcaa 1440
   gacgatagtt accggataag gcgcagcggt cgggctgaac ggggggttcg tgcacacagc 1500
   ccagcttgga gcgaacgacc tacaccgaac tgagatacct acagcgtgag ctatgagaaa 1560
   gcgccacgct tcccgaaggg agaaaggcgg acaggtatcc ggtaagcggc agggtcggaa 1620
   caggagagcg cacgagggag cttccagggg gaaacgcctg gtatctttat agtcctgtcg 1680
   ggtttcgcca cctctgactt gagcgtcgat ttttgtgatg ctcgtcaggg gggcggagcc 1740
   tatggaaaaa cgccagcaac gcggcctttt tacggttcct ggccttttgc tggccttttg 1800
   ctcacatgtt ctttcctgcg ttatcccctg attctgtgga taaccgtatt accgcctttg 1860
   agtgagctga taccgctcgc cgcagccgaa cgaccgagcg cagcgagtca gtgagcgagg 1920
   aagcggaaga gcgcccaata cgcaaaccgc ctctccccgc gcgttggccg attcattaat 1980
   gcagctggca cgacaggttt cccgactgga aagcgggcag tgagcgcaac gcaattaatg 2040
   tgagttagct cactcattag gcaccccagg ctttacactt tatgcttccg gctcgtatgt 2100
   tgtgtggaat tgtgagcgga taacaatttc acacaggaaa cagctatgac catgattacg 2160
   ccaagcgcgc aattaaccct cactaaaggg aacaaaagct ggagctccac cgcggtggcg 2220
   gccgctctag ttggatttgc tcccccgccg tcgttcaatg agaatggata agaggctcgt 2280
   gggattgacg tgagggggca gggatggcta tatttctggg agcgaactcc gggcgaattt 2340
   gaagcgcttg gatacagttg tagggaggga tccatggctc gtgaagcggt tatcgccgaa 2400
   gtatcaactc aactatcaga ggtagttggc gtcatcgagc gccatctcga accgacgttg 2460
   ctggccgtac atttgtacgg ctccgcagtg gatggcggcc tgaagccaca cagtgatatt 2520
   gatttgctgg ttacggtgac cgtaaggctt gatgaaacaa cgcggcgagc tttgatcaac 2580
   gaccttttgg aaacttcggc ttcccctgga gagagcgaga ttctccgcgc tgtagaagtc 2640
   accattgttg tgcacgacga catcattccg tggcgttatc cagctaagcg cgaactgcaa 2700
   tttggagaat ggcagcgcaa tgacattctt gcaggtatct tcgagccagc cacgatcgac 2760
   attgatctgg ctatcttgct gacaaaagca agagaacata gcgttgcctt ggtaggtcca 2820
   gcggcggagg aactctttga tccggttcct gaacaggatc tatttgaggc gctaaatgaa 2880
   accttaacgc tatggaactc gccgcccgac tgggctggcg atgagcgaaa tgtagtgctt 2940
   acgttgtccc gcatttggta cagcgcagta accggcaaaa tcgcgccgaa ggatgtcgct 3000
   gccgactggg caatggagcg cctgccggcc cagtatcagc ccgtcatact tgaagctaga 3060
   caggcttatc ttggacaaga agaagatcgc ttggcctcgc gcgcagatca gttggaagaa 3120
   tttgtccact acgtgaaagg cgagatcact aaggtagttg gcaaataact gcaggatcct 3180
   ggcctagtct ataggaggtt ttgaaaagaa aggagcaata atcattttct tgttctatca 3240
   agagggtgct attgctcctt tctttttttc ttttatttta tttactagta ttttacttac 3300
   atagactttt ttgtttacat tatagaaaaa gaaggagagg ttattttctt gcatttattc 3360
   atgattgagt attctatttt gattttgtat ttgtttaaaa ttgtagaaat agaacttgtt 3420
   tctcttcttg ctaatgttac tatatctttt tgattttttt tttccaaaaa aaaatcaaat 3480
   tttgacttct tcttatctct tatctttgaa tatctcttat ctttgaaata ataatatcat 3540
   tgaaataaga aagaagagct atattcgaag cttctacata caccttggtt gacacgagta 3600
   tataagtcat gttatactgt tgaataacaa gccttccatt ttctattttg atttgtagaa 3660
   aactagtgtg cttgggagtc cctgatgatt aaataaacca agatctaaaa ggagaaatta 3720
   agcatgctct agatcgatga attcgccctt ccgaagcttg aaattcaatt aaggaaataa 3780
   attaaggaaa tacaaaaagg ggggtagtca tttgtatata actttgtatg acttttctct 3840
   tctatttttt tgtatttcct ccctttcctt ttctatttgt atttttttat cattgcttcc 3900
   attgaattac tagtcgacct cgaggggggg cccggtaccc aattcgccct atagtgagtc 3960
   gtattacgcg cgctcactgg ccgtcgtttt acaacgtcgt gactgggaaa accctggcgt 4020
   tacccaactt aatcgccttg cagcacatcc ccctttcgcc agctggcgta atagcgaaga 4080
   ggcccgcacc gatcgccctt cccaacagtt gcgcagcctg aatggcgaat gggacgcgcc 4140
   ctgtagcggc gcattaagcg cggcgggtgt ggtggttacg cgcagcgtga ccgctacact 4200
   tgccagcgcc ctagcgcccg ctcctttcgc tttcttccct tcctttctcg ccacgttcgc 4260
   cggctttccc cgtcaagctc taaatcgggg gctcccttta gggttccgat ttagtgcttt 4320
   acggcacctc gaccccaaaa aacttgatta gggtgatggt tcacgtagtg ggccatcgcc 4380
   ctgatagacg gtttttcgcc ctttgacgtt ggagtccacg ttctttaata gtggactctt 4440
   gttccaaact ggaacaacac tcaaccctat ctcggtctat tcttttgatt tataagggat 4500
   tttgccgatt tcggcctatt ggttaaaaaa tgagctgatt taacaaaaat ttaacgcgaa 4560
   ttttaacaaa atattaacgc ttacaattta g 4591
<210> 11
   <211> 51
   <212> DNA
   <213> Artificial sequence
<220>
   <223> multi-cloning regions
<400> 11
   ccaagatcta aaaggagaaa ttaagcatgc tctagatcga tgaattcgcc c 51
<210> 12
   <211> 142
   <212> DNA
   <213> Nicotiana tabacum
<220>
   <223> rrn promoter
<400> 12
   ctagttggat ttgctccccc gccgtcgttc aatgagaatg gataagaggc tcgtgggatt 60
   gacgtgaggg ggcagggatg gctatatttc tgggagcgaa ctccgggcga atttgaagcg 120
   cttggataca gttgtaggga gg 142
<210> 13
   <211> 390
   <212> DNA
   <213> Nicotiana tabacum
<220>
   <223> psbA terminator
<400> 13
   gatcctggcc tagtctatag gaggttttga aaagaaagga gcaataatca ttttcttgtt 60
   ctatcaagag ggtgctattg ctcctttctt tttttctttt tatttattta ctagtatttt 120
   acttacatag acttttttgt ttacattata gaaaaagaag gagaggttat tttcttgcat 180
   ttattcatga ttgagtattc tattttgatt ttgtatttgt ttaaaattgt agaaatagaa 240
   cttgtttctc ttcttgctaa tgttactata tctttttgat tttttttttc caaaaaaaaa 300
   tcaaattttg acttcttctt atctcttatc tttgaatatc tcttatcttt gaaataataa 360
   tatcattgaa ataagaaaga agagctatat 390
<210> 14
   <211> 5581
   <212> DNA
   <213> Artificial sequence
<220>
   <223> pLD200
<400> 14
   tcgcgcgttt cggtgatgac ggtgaaaacc tctgacacat gcagctcccg gagacggtca    60
   cagcttgtct gtaagcggat gccgggagca gacaagcccg tcagggcgcg tcagcgggtg   120
   ttggcgggtg tcggggctgg cttaactatg cggcatcaga gcagattgta ctgagagtgc   180
   accatatgcg gtgtgaaata ccgcacagat gcgtaaggag aaaataccgc atcaggcgcc   240
   attcgccatt caggctgcgc aactgttggg aagggcgatc ggtgcgggcc tcttcgctat   300
   tacgccagct ggcgaaaggg ggatgtgctg caaggcgatt aagttgggta acgccagggt   360
   tttcccagtc acgacgttgt aaaacgacgg ccagtgaatt catgagttgt agggagggat   420
   ttatgtcacc acaaacagag actaaagcaa gtgttggatt caaagctggt gttaaagagt   480
   acaaattgac ttattatact cctgagtacc aaaccaagga tactgatata ttggcagcat   540
   tccgagtaac tcctcaacct ggagttccac ctgaagaagc aggggccgcg gtagctgccg   600
   aatcttctac tggtacatgg acaactgtat ggaccgatgg acttaccagc cttgatcgtt   660
   acaaagggcg atgctaccgc atcgagcgtg ttgttggaga aaaagatcaa tatattgctt   720
   atgtagctta ccctttagac ctttttgaag aaggttctgt taccaacatg tttacttcca   780
   ttgtaggtaa cgtatttggg ttcaaagccc tgcgcgctct acgtctggaa gatctgcgaa   840
   tccctcctgc ttatgttaaa actttccaag gtccgcctca tgggatccaa gttgaaagag   900
   ataaattgaa caagtatggt cgtcccctgt tgggatgtac tattaaacct aaattggggt   960
   tatctgctaa aaactacggt agagccgttt atgaatgtct tcgcggtgga cttgatttta  1020
   ctaaagatga tgagaacgtg aactcacaac catttatgcg ttggagagat cgtttcttat  1080
   tttgtgccga agcactttat aaagcacagg ctgaaacagg tgaaatcaaa gggcattact  1140
   tgaatgctac tgcaggtaca tgcgaagaaa tgatcaaaag agctgtattt gctagagaat  1200
   tgggcgttcc gatcgtaatg catgactact taacgggggg attcaccgca aatactagct  1260
   tggctcatta ttgccgagat aatggtctac ttcttcacat ccaccgtgca atgcatgcgg  1320
   ttattgatag acagaagaat catggtatcc acttccgggt attagcaaaa gcgttacgta  1380
   tgtctggtgg agatcatatt cactctggta ccgtagtagg taaacttgaa ggtgaaagag  1440
   acataacttt gggctttgtt gatttactgc gtgatgattt tgttgaacaa gatcgaagtc  1500
   gcggtattta tttcactcaa gattgggtct ctttaccagg tgttctaccc gtggcttcag  1560
   gaggtattca cgtttggcat atgcctgctc tgaccgagat ctttggggat gattccgtac  1620
   tacagttcgg tggaggaact ttaggacatc cttggggtaa tgcgccaggt gccgtagcta  1680
   atcgagtagc tctagaagca tgtgtaaaag ctcgtaatga aggacgtgat cttgctcagg  1740
   aaggtaatga aattattcgc gaggcttgca aatggagccc ggaactagct gctgcttgtg  1800
   aagtatggaa agagatcgta tttaattttg cagcagtgga cgttttggat aagtaaaaac 1860
   agtagacatt agcagataaa ttagcaggaa ataaagaagg ataaggagaa agaactcaag 1920
   taattatcct tcgttctctt aattgaattg caattaaact cggcccaatc ttttactaaa 1980
   aggattgagc cgaatacaac aaagattcta ttgcatatat tttgactaag tatatactta 2040
   cctagatata caagatttga aatacaaaat ctagaaaact aaatcaaaat ctaagactca 2100
   aatctttcta ttgttgtctt ggatcgcggc cgcgctagcg tcgacgatcc ttaggattgg 2160
   tatattcttt tctatcctgt agtttgtagt ttccctgaat caagccaagt atcacacctc 2220
   tttctaccca tcctgtatat tgtccccttt gttccgtgtt gaaatagaac cttaatttat 2280
   tacttatttt tttattaaat tttagatttg ttagtgatta gatattagta ttagacgaga 2340
   ttttacgaaa caattatttt tttatttctt tataggagag gacaaatctc ttttttcgat 2400
   gcgaatttga cacgacatag gagaagccgc cctttattaa aaattatatt attttaaata 2460
   atataaaggg ggttccaaca tattaatata tagtgaagtg ttcccccaga ttcagaactt 2520
   tttttcaata ctcacaatcc ttattagtta ataatcctag tgattggatt tctatgctta 2580
   gtctgatagg aaataagata ttcaaataaa taattttata gcgaatgact attcatctat 2640
   tgtattttca tgcaaatagg gggcaagaaa actctatgga aagatggtgg tttaattcga 2700
   tgttgtttaa gaaggagttc gaacgcaggt gtgggctaaa taaatcaatg ggcagtcttg 2760
   gtcctattga aaataccaat gaagatccaa atcgaaaagt gaaaaacatt catagttgga 2820
   ggaatcgtga caattctagt tgcagtaatg ttgattattt attcggcgtt aaagacattc 2880
   ggaatttcat ctctgatgac acttttttag ttagtgatag gaatggagac agttattcca 2940
   tctattttga tattgaaaat catatttttg agattgacaa cgatcattct tttctgagtg 3000
   aactagaaag ttctttttat agttatcgaa actcgaatta tcggaataat ggatttaggg 3060
   gcgaagatcc ctactataat tcttacatgt atgatactca atatagttgg aataatcaca 3120
   ttaatagttg cattgatagt tatcttcagt ctcaaatctg tatagatact tccattataa 3180
   gtggtagtga gaattacggt gacagttaca tttatagggc cgtttgtggt ggtgaaagtc 3240
   gaaatagtag tgaaaacgag ggttccagta gacgaactcg cacgaagggc agtgatttaa 3300
   ctataagaga aagttctaat gatctcgacc tgcaggcatg caagcttggc gtaatcatgg 3360
   tcatagctgt ttcctgtgtg aaattgttat ccgctcacaa ttccacacaa catacgagcc 3420
   ggaagcataa agtgtaaagc ctggggtgcc taatgagtga gctaactcac attaattgcg 3480
   ttgcgctcac tgcccgcttt ccagtcggga aacctgtcgt gccagctgca ttaatgaatc 3540
   ggccaacgcg cggggagagg cggtttgcgt attgggcgct cttccgcttc ctcgctcact 3600
   gactcgctgc gctcggtcgt tcggctgcgg cgagcggtat cagctcactc aaaggcggta 3660
   atacggttat ccacagaatc aggggataac gcaggaaaga acatgtgagc aaaaggccag 3720
   caaaaggcca ggaaccgtaa aaaggccgcg ttgctggcgt ttttccatag gctccgcccc 3780
   cctgacgagc atcacaaaaa tcgacgctca agtcagaggt ggcgaaaccc gacaggacta 3840
   taaagatacc aggcgtttcc ccctggaagc tccctcgtgc gctctcctgt tccgaccctg 3900
   ccgcttaccg gatacctgtc cgcctttctc ccttcgggaa gcgtggcgct ttctcaatgc 3960
   tcacgctgta ggtatctcag ttcggtgtag gtcgttcgct ccaagctggg ctgtgtgcac 4020
   gaaccccccg ttcagcccga ccgctgcgcc ttatccggta actatcgtct tgagtccaac 4080
   ccggtaagac acgacttatc gccactggca gcagccactg gtaacaggat tagcagagcg 4140
   aggtatgtag gcggtgctac agagttcttg aagtggtggc ctaactacgg ctacactaga 4200
   aggacagtat ttggtatctg cgctctgctg aagccagtta ccttcggaaa aagagttggt 4260
   agctcttgat ccggcaaaca aaccaccgct ggtagcggtg gtttttttgt ttgcaagcag 4320
   cagattacgc gcagaaaaaa aggatctcaa gaagatcctt tgatcttttc tacggggtct 4380
   gacgctcagt ggaacgaaaa ctcacgttaa gggattttgg tcatgagatt atcaaaaagg 4440
   atcttcacct agatcctttt aaattaaaaa tgaagtttta aatcaatcta aagtatatat 4500
   gagtaaactt ggtctgacag ttaccaatgc ttaatcagtg aggcacctat ctcagcgatc 4560
   tgtctatttc gttcatccat agttgcctga ctccccgtcg tgtagataac tacgatacgg 4620
   gagggcttac catctggccc cagtgctgca atgataccgc gagacccacg ctcaccggct 4680
   ccagatttat cagcaataaa ccagccagcc ggaagggccg agcgcagaag tggtcctgca 4740
   actttatccg cctccatcca gtctattaat tgttgccggg aagctagagt aagtagttcg 4800
   ccagttaata gtttgcgcaa cgttgttgcc attgctacag gcatcgtggt gtcacgctcg 4860
   tcgtttggta tggcttcatt cagctccggt tcccaacgat caaggcgagt tacatgatcc 4920
   cccatgttgt gcaaaaaagc ggttagctcc ttcggtcctc cgatcgttgt cagaagtaag 4980
   ttggccgcag tgttatcact catggttatg gcagcactgc ataattctct tactgtcatg 5040
   ccatccgtaa gatgcttttc tgtgactggt gagtactcaa ccaagtcatt ctgagaatag 5100
   tgtatgcggc gaccgagttg ctcttgcccg gcgtcaatac gggataatac cgcgccacat 5160
   agcagaactt taaaagtgct catcattgga aaacgttctt cggggcgaaa actctcaagg 5220
   atcttaccgc tgttgagatc cagttcgatg taacccactc gtgcacccaa ctgatcttca 5280
   gcatctttta ctttcaccag cgtttctggg tgagcaaaaa caggaaggca aaatgccgca 5340
   aaaaagggaa taagggcgac acggaaatgt tgaatactca tactcttcct ttttcaatat 5400
   tattgaagca tttatcaggg ttattgtctc atgagcggat acatatttga atgtatttag 5460
   aaaaataaac aaataggggt tccgcgcaca tttccccgaa aagtgccacc tgacgtctaa 5520
   gaaaccatta ttatcatgac attaacctat aaaaataggc gtatcacgag gccctttcgt 5580
   c                                                                 5581
<210> 15
   <211> 1434
   <212> DNA
   <213> Nicotiana tabacum
<220>
   <223> rbcL
<400> 15
<210> 16
   <211> 705
   <212> DNA
   <213> Nicotiana tabacum
<220>
   <223> accD
<400> 16
   aatgactatt catctattgt attttcatgc aaataggggg caagaaaact ctatggaaag      60
   atggtggttt aattcgatgt tgtttaagaa ggagttcgaa cgcaggtgtg ggctaaataa     120
   atcaatgggc agtcttggtc ctattgaaaa taccaatgaa gatccaaatc gaaaagtgaa     180
   aaacattcat agttggagga atcgtgacaa ttctagttgc agtaatgttg attatttatt     240
   cggcgttaaa gacattcgga atttcatctc tgatgacact tttttagtta gtgataggaa     300
   tggagacagt tattccatct attttgatat tgaaaatcat atttttgaga ttgacaacga     360
   tcattctttt ctgagtgaac tagaaagttc tttttatagt tatcgaaact cgaattatcg     420
   gaataatgga tttaggggcg aagatcccta ctataattct tacatgtatg atactcaata     480
   tagttggaat aatcacatta atagttgcat tgatagttat cttcagtctc aaatctgtat     540
   agatacttcc attataagtg gtagtgagaa ttacggtgac agttacattt atagggccgt     600
   ttgtggtggt gaaagtcgaa atagtagtga aaacgagggt tccagtagac gaactcgcac     660
   gaagggcagt gatttaacta taagagaaag ttctaatgat ctcga                     705
<210> 17
   <211> 21
   <212> DNA
   <213> Artificial sequence
<220>
   <223> polylinker
<400> 17
   cgcggccgcg ctagcgtcga c 21
<210> 18
   <211> 7
   <212> DNA
   <213> Artificial sequence
<220>
   <223> Shine-Dalgarno sequence
<400> 18
   aggaggu 7